(19) Europäisches Patentamt
European Patent Office
Office européen des brevets

(11) Veröffentlichungsnummer: **0 096 786**
**B1**

# (12) EUROPÄISCHE PATENTSCHRIFT

(45) Veröffentlichungstag der Patentschrift: **16.09.87**

(21) Anmeldenummer: **83105313.7**

(22) Anmeldetag: **30.05.83**

(51) Int. Cl.[4]: **C 07 D 249/08**, C 07 D 233/60, A 01 N 43/64, A 01 N 43/50 // C07D303/08, C07C49/80, C07C33/28

(54) **Hydroxyalkinyl-azolyl-Derivate, Verfahren zu ihrer Herstellung sowie ihre Verwendung als Fungizide.**

(30) Priorität: **12.06.82 DE 3222191**

(43) Veröffentlichungstag der Anmeldung: **28.12.83 Patentblatt 83/52**

(45) Bekanntmachung des Hinweises auf die Patenterteilung: **16.09.87 Patentblatt 87/38**

(84) Benannte Vertragsstaaten: **AT BE CH DE FR GB IT LI NL SE**

(56) Entgegenhaltungen:
**EP - A - 0 033 501**
**EP - A - 0 052 424**
**EP - A - 0 071 009**

**Die Akte enthält technische Angaben, die nach dem Eingang der Anmeldung eingereicht wurden und die nicht in dieser Patentschrift enthalten sind.**

(73) Patentinhaber: **BAYER AG, Konzernverwaltung RP Patentabteilung, D-5090 Leverkusen 1 Bayerwerk (DE)**

(72) Erfinder: **Jäger, Gerhard, Dr., Gellertstrasse 18, D-5090 Leverkusen 1 (DE)**
Erfinder: **Böckmann, Klaus, Dr., Andreas-Gryphius-Strasse 7, D-5000 Köln 80 (DE)**
Erfinder: **Büchel, Karl Heinz, Prof. Dr., Dabringhausener Strasse 42, D-5093 Burscheid (DE)**
Erfinder: **Brandes, Wilhelm, Dr., Eichendorffstrasse 3, D-5653 Leichlingen (DE)**
Erfinder: **Frohberger, Paul-Ernst, Dr., Willi-Baumeister-Strasse 5, D-5090 Leverkusen 1 (DE)**
Erfinder: **Scheinpflug, Hans, Dr., Am Thelenhof 15, D-5090 Leverkusen 1 (DE)**

**EP 0 096 786 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

**Beschreibung**

Die Erfindung betrifft neue Hydroxyalkinylazolyl-Derivate, mehrere Verfahren zu ihrer Herstellung sowie ihre Verwendung als Fungizide.

Es ist bereits bekannt geworden, dass bestimmte Hydroxyalkyl-triazole, wie beispielsweise
2-(4-Biphenylyl)-1-(2,3-dichlorphenyl)-3-(1,2,4-triazol-1-yl)-2-propanol oder
2-(4-Biphenylyl)-1-(4-chlorphenyl)-3-(1,2,4-triazol-1-yl)-2-propanol,
fungizide Eigenschaften aufweisen (vergleiche DE-A 29 20 374). Die Wirksamkeit dieser Verbindungen ist jedoch, insbesondere bei niedrigen Aufwandmengen und -konzentrationen, nicht immer ganz befriedigend.

Aus der EP-A 0 025 516 sind Hydroxyalkinyl-azolyl-Derivate bekannt, in denen das Carbinol-Kohlenstoffatom mit einem gegebenenfalls durch Halogen substituierten Alkylrest verknüpft sein kann. Es werden jedoch keine Verbindungen offenbart, in denen der betreffende Rest für halogeniertes tert.-Butyl oder für andere Alkylreste als tert.-Butyl steht.

Ferner werden in der US-A 4 413 003 Hydroxyalkinyl-imidazolyl-Derivate beschrieben, welche einen gegebenenfalls substituierten Phenylrest am zentralen Kohlenstoffatom enthalten. Entsprechende Stoffe, in denen andere Reste als gegebenenfalls substituiertes Phenyl an der betreffenden Stelle vorhanden ist, werden allerdings nicht erwähnt.

Die EP-A. 0 033 501 ist auf fungizid wirksame Imidazolylalkohole gerichtet, in denen ein Allyl-Rest enthalten ist. Analoge Verbindungen mit einem Propargyl-Substituenten werden nicht aufgeführt.

In den Verbindungen, die in der EP-A 0 052 424 offenbart werden, kann das zentrale Kohlenstoffatom direkt mit einer $-C\equiv C$-Gruppe verbunden sein. Es werden aber keine Substanzen beschrieben, die zwischen der $-C\equiv C$-Gruppe und dem Carbinol-Kohlenstoffatom noch eine $CH_2$-Einheit aufweisen.

Schliesslich sind aus der EP-A 0 071 009 Alkinylazol-Derivate bekannt, in denen das zum Azolylrest benachbarte Kohlenstoffatom einen gegebenenfalls substituierten Aryl-Rest oder einen gegebenenfalls substituierten Aryloxy-Rest trägt. Andere als die zuvor genannten Reste sind an der betreffenden Stelle des Moleküls jedoch nicht vorgesehen.

Es wurden neue Hydroxyalkinyl-azolyl-Derivate der allgemeinen Formel I

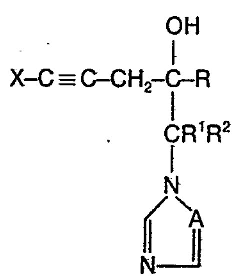

(I)

in welcher

A für ein Stickstoffatom oder eine CH-Gruppe steht,

R für gegebenenfalls einfach bis dreifach, gleich oder verschieden durch Alkyl mit 1 bis 4 Kohlenstoffatomen substituiertes Cycloalkyl mit 3 bis 7 Kohlenstoffatomen, für Adamantyl und

R ferner für die Gruppierungen

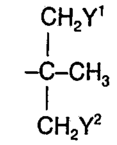

und

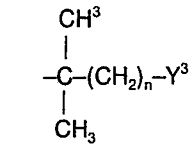

steht, wobei

$Y^1$ für Wasserstoff oder Halogen steht,

$Y^2$ für Halogen steht,

$Y^3$ für Alkyl mit 2 bis 4 Kohlenstoffatomen, Alkoxy und Alkylthio mit jeweils 1 bis 4 Kohlenstoffatomen, Halogenalkoxy und Halogenalkylthio mit jeweils 1 bis 2 Kohlenstoff- und 1 bis 5 gleichen oder verschiedenen Halogenatomen, Alkenyl mit 2 bis 6 Kohlenstoffatomen, Alkoxycarbonyl mit 1 bis 4 Kohlenstoffatomen im Alkoxyteil, Cyano sowie für gegebenenfalls einfach bis dreifach, gleich oder verschieden substituiertes Phenyl, Phenoxy, Phenylthio, Phenylalkoxy mit 1 bis 4 Kohlenstoffatomen im Alkoxyteil und Phenylalkylthio mit 1 bis 4 Kohlenstoffatomen im Alkylthioteil steht, wobei jeweils als Phenylsubstituenten genannt seien: Halogen, Alkyl mit 1 bis 4 Kohlenstoffatomen; Alkoxy und Alkylthio mit jeweils 1 bis 2 Kohlenstoffatomen; Halogenalkyl, Halogenalkoxy und Halogenalkylthio mit jeweils 1 bis 2 Kohlenstoff- und 1 bis 5 gleichen oder verschiedenen Halogenatomen, Cyclohexyl, Dialkylamino mit 1 bis 4 Kohlenstoffatomen in jedem Alkylteil, Nitro, Cyano, sowie Alkoxycarbonyl mit 1 bis 4 Kohlenstoffatomen im Alkoxyteil; und gegebenenfalls durch Halogen substituiertes Phenyl;

n für die Zahlen 0, 1 oder 2 steht;

$R^1$ für Wasserstoff, geradkettiges oder verzweigtes Alkyl mit 1 bis 4 Kohlenstoffatomen, Alkenyl und Alkinyl mit jeweils 2 bis 4 Kohlenstoffatomen sowie für gegebenenfalls einfach bis dreifach, gleich oder verschieden substituiertes Benzyl, wobei als Phenylsubstituenten die bei $Y^3$ bereits genannten Phenylsubstituenten infrage kommen;

$R^2$ für Wasserstoff oder Methyl steht und

X für Wasserstoff, Brom oder Iod steht,

sowie deren Säureadditions-Salze und Metallsalz-Komplexe gefunden.

Weiterhin wurde gefunden, dass man die Hydroxyalkinylazolyl-Derivate der allgemeinen Formel I sowie deren Säureadditions-Salze und Metallsalz-Komplexe erhält, wenn man

a) Azolyl-ketone der allgemeinen Formel II

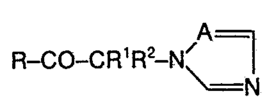

(II)

in welcher

A, R, $R^1$ und $R^2$ die oben angegebene Bedeutung haben, mit Propargylhalogeniden der allgemeinen Formel III

$$HC{\equiv}C{-}CH_2{-}Hal \qquad (III)$$

in welcher
Hal für Chlor oder Brom steht,
in Gegenwart von aktiviertem Aluminium und eines Verdünnungsmittels umsetzt, oder

b) Hydroxyalkinyl-halogenide der allgemeinen Formel IV

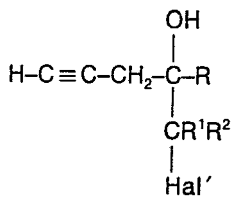

(IV)

in welcher
R, $R^1$ und $R^2$ die oben angegebene Bedeutung haben und
Hal' für Chlor oder Brom steht,
mit Azolen der allgemeinen Formel V

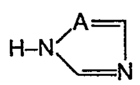

(V)

in welcher
A die oben angegebene Bedeutung hat,
in Gegenwart einer Base und eines Verdünnungsmittels umsetzt, oder

c) Oxirane der allgemeinen Formel VI

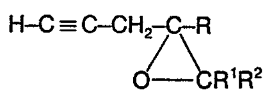

(VI)

in welcher
R, $R^1$ und $R^2$ die oben angegebene Bedeutung haben,
mit Azolen der allgemeinen Formel V

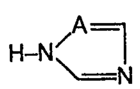

(V)

in welcher
A die oben angegebene Bedeutung hat,
in Gegenwart eines Verdünnungsmittels und gegebenenfalls in Gegenwart einer Base umsetzt, oder

d) gegebenenfalls die nach den Verfahren (a), (b) oder (c) erhaltenen Hydroxyalkinyl-azolyl-Derivate der allgemeinen Formel Ia

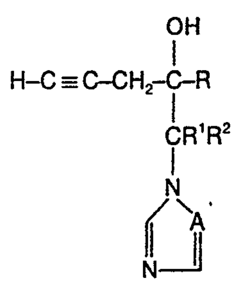

(Ia)

in welcher
A, R, $R^1$ und $R^2$ die oben angegebene Bedeutung haben,
in an sich bekannter Art und Weise mit Alkalihypohalogenit umsetzt, und gegebenenfalls noch an die so erhaltenen Verbindungen der allgemeinen Formel I eine Säure oder ein Metallsalz addiert.

Schliesslich wurde gefunden, dass die neuen Hydroxyalkinylazolyl-Derivate der allgemeinen Formel I sowie deren Säureadditions-Salze und Metallsalz-Komplexe starke fungizide Eigenschaften aufweisen. Dabei zeigen die erfindungsgemässen Verbindungen überraschenderweise eine bessere fungizide Wirksamkeit als die aus dem Stand der Technik bekannten Hydroxyalkyl-triazole, wie beispielsweise
2-(4-Biphenylyl)-1-(2,4-dichlorphenyl)-3-(1,2,4-triazol-1-yl)-2-propanol oder
2-(4-Biphenylyl)-1-(4-chlorphenyl)-3-(1,2,4-triazol-1-yl)-2-propanol,
welches chemisch ähnliche Verbindungen sind. Die erfindungsgemässen Wirkstoffe stellen somit eine Bereicherung der Technik dar.

Ausserdem sind die neuen Hydroxyalkinylazolyl-Derivate interessante Zwischenprodukte. So können z. B. die Verbindungen der allgemeinen Formel I an der Hydroxy-Gruppe in üblicher Weise in die entsprechenden Ether überführt werden. Weiterhin können durch Umsetzung mit z. B. Acylhalogeniden, Isocyanaten oder Carbamoylchloriden in prinzipiell bekannter Weise Acyl- oder Carbamoyl-Derivate der Verbindungen der allgemeinen Formel I erhalten werden.

Die erfindungsgemässen Hydroxyalkinylazolyl-Derivate sind durch die Formel I allgemein definiert.

Bevorzugt sind diejenigen Verbindungen der allgemeinen Formel I, in denen
R für jeweils einfach oder zweifach, gleich oder verschieden, durch Methyl, Ethyl, Isopropyl oder tert.-Butyl substituiertes Cyclopropyl, Cyclopentyl oder Cyclohexyl steht, für Adamantyl steht und
R ferner für die Gruppierungen

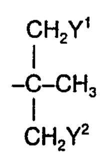

und

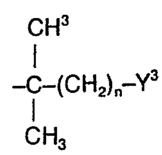

steht, wobei
$Y^1$ für Wasserstoff, Fluor, Chlor oder Brom steht,
$Y^2$ für Fluor, Chlor oder Brom steht,
$Y^3$ für Ethyl, n-Propyl, Isopropyl, tert.-Butyl, Methoxy, Ethoxy, Methylthio, Ethylthio, Trifluormethoxy, Trifluormethylthio, Vinyl, Methoxycarbonyl, Ethoxycarbonyl, Cyano, sowie für gegebenenfalls einfach bis zweifach, gleich oder verschieden substituiertes Phenyl, Phenoxy, Phenylthio, Phenylmethoxy und Phenylmethylthio steht, wobei jeweils als Phenylsubstituenten genannt seien: Fluor, Chlor, Brom, Methyl, Ethyl, Isopropyl, tert.-Butyl, Methoxy, Methylthio, Ethoxy, Trifluormethyl, Trifluormethoxy, Trifluormethylthio, Nitro, Dimethylamino, Methoxycarbonyl, sowie gegebenenfalls durch Chlor substituiertes Phenyl,
n für die Zahlen 0, 1 oder 2 steht,

$R^1$ für Wasserstoff, Methyl, Vinyl, Allyl, Propargyl sowie für gegebenenfalls einfach bis zweifach, gleich oder verschieden, substituiertes Benzyl steht, wobei als Phenylsubstituenten vorzugsweise die bei $Y^3$ bereits vorzugsweise genannten Phenylsubstituenten infrage kommen, und

A, $R^2$ und X für die in der Erfindungsdefinition angegebenen Bedeutungen stehen.

Bevorzugte erfindungsgemässe Verbindungen sind auch Additionsprodukte aus Säuren und denjenigen Hydroxyalkinyl-azolyl-Derivaten der allgemeinen Formel I, in denen die Substituenten A, R, $R^1$, $R^2$ und X die Bedeutungen haben, die bereits als bevorzugt für diese Substituenten genannt wurden.

Zu den Säuren, die addiert werden können, gehören vorzugsweise Halogenwasserstoffsäuren, wie z.B. die Chlorwasserstoffsäure und die Bromwasserstoffsäure, insbesondere die Chlorwasserstoffsäure, ferner Phosphorsäure, Salpetersäure, mono- und bifunktionelle Carbonsäuren und Hydroxycarbonsäuren, wie z.B. Essigsäure, Maleinsäure, Bernsteinsäure, Fumarsäure, Weinsäure, Citronensäure, Salicylsäure, Sorbinsäure und Milchsäure, sowie Sulfonsäuren, wie z.B. p-Toluolsulfonsäure und 1,5-Naphthalindisulfonsäure.

Ausserdem bevorzugte erfindungsgemässe Verbindungen sind Additionsprodukte aus Salzen von Metallen der II. bis IV. Haupt- und der I. und II. sowie IV. bis VIII. Nebengruppe und denjenigen Hydroxyalkinyl-azolyl-Derivaten der allgemeinen Formel I, in denen die Substituenten A, R, $R^1$, $R^2$ und X die Bedeutungen haben, die bereits als bevorzugt für diese Substituenten genannt wurden.

Hierbei sind Salze des Kupfers, Zinks, Mangans, Magnesiums, Zinns, Eisens und des Nickels besonders bevorzugt. Als Anionen dieser Salze kommen solche in Betracht, die sich von solchen Säuren ableiten, die zu physiologisch verträglichen Additionsprodukten führen. Besonders bevorzugte derartige Säuren sind in diesem Zusammenhang die Halogenwasserstoffsäuren, wie z.B. die Chlorwasserstoffsäure und die Bromwasserstoffsäure, ferner Phosphorsäure, Salpetersäure und Schwefelsäure.

Verwendet man beispielsweise

5-(2,4-Dichlorphenoxy)-3,3-dimethyl-1-(1,2,4-triazol-1-yl)-2-pentanon

und Propargylbromid in Gegenwart von Aluminium und katalytischen Mengen Quecksilber(II)-chlorid als Ausgangsstoffe, so kann der Ablauf des erfindungsgemässen Verfahrens (a) durch das folgende Formelschema wiedergegeben werden:

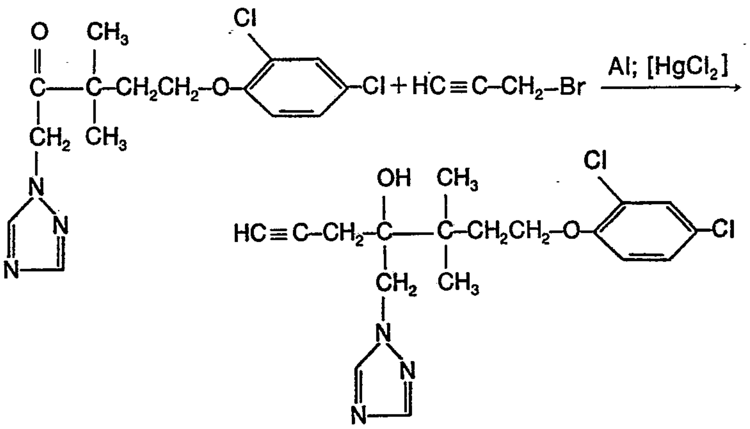


Die bei der Durchführung des erfindungsgemässen Verfahrens (a) als Ausgangsstoffe zu verwendenden Azolyl-ketone sind durch die Formel II allgemein definiert. In dieser Formel haben A, R, $R^1$ und $R^2$ diejenigen Bedeutungen, die bereits im Zusammenhang mit der Beschreibung der erfindungsgemässen Stoffe der allgemeinen Formel I für diese Substituenten genannt wurden.

Die Azolyl-ketone der allgemeinen Formel II sind weitgehend bekannt (vergleiche z.B. DE-A 24 31 407, DE-A 26 10 022, DE-A 26 38 470, DE-A 29 51 164, DE-A 30 48 266, DE-A 31 45 857 und DE-A 31 45 858) bzw. können in allgemein üblicher Art und Weise erhalten werden.

Die ausserdem für das erfindungsgemässe Verfahren (a) als Ausgangsstoffe zu verwendenden Propargylhalogenide der allgemeinen Formel III sind allgemein bekannte Verbindungen der organischen Chemie.

Die bei der Durchführung des erfindungsgemässen Verfahrens (b) als Ausgangsstoffe zu verwendenden Hydroxyalkinylhalogenide sind durch die Formel IV allgemein definiert. In dieser Formel haben R, $R^1$ und $R^2$ die Bedeutungen, die bereits im Zusammenhang mit der Beschreibung der erfindungsgemässen Stoffe der allgemeinen Formel I für diese Substituenten genannt wurden.

Die Hydroxyalkinyl-halogenide der allgemeinen Formel IV sind noch nicht bekannt; sie können jedoch in einfacher Weise erhalten werden, indem man Halogenketone der allgemeinen Formel VII

$$R\text{–}CO\text{–}CR^1R^2\text{–}Hal' \qquad (VII)$$

in welcher

Hal', R, $R^1$ und $R^2$ die oben angegebene Bedeutung haben,

gemäss Verfahren (a) mit Propargylhalogeniden der allgemeinen Formel III umsetzt.

Die Halogenketone der allgemeinen Formel VII sind bekannt; bzw. können in allgemein bekannter Art und Weise erhalten werden.

Die bei der Durchführung des erfindungsgemässen Verfahrens (c) als Ausgangsstoffe zu verwendenden Oxirane sind durch die Formel VI allgemein definiert. In dieser Formel haben R, $R^1$ und $R^2$ die Bedeutungen, die bereits im Zusammenhang mit der Beschreibung der erfindungsgemässen Stoffe der allgemeinen Formel I für diese Substituenten genannt wurden.

Die Oxirane der allgemeinen Formel VI sind noch nicht bekannt; sie können jedoch in einfacher Weise erhalten werden, indem man Hydroxyalkinyl-halogenide der allgemeinen Formel IV mit einer Base, wie beispielsweise Kaliumcarbonat, in einem Zweiphasensystem, wie beispielsweise Methylenchlorid/Wasser, in Gegenwart eines Phasentransferkatalysators, wie beispielsweise Triethylbenzylammoniumchlorid, bei Temperaturen zwischen 20 und 50 °C umsetzt.

Die ausserdem für die erfindungsgemässen Verfahren (b) und (c) als Ausgangsstoffe zu verwendenden Azole sind durch die Formel V allgemein definiert. In dieser Formel steht A für die Bedeutungen, die bereits in der Erfindungsdefinition genannt wurden.

Die Azole der allgemeinen Formel V sind allgemein bekannte Verbindungen der organischen Chemie.

Die für das erfindungsgemässe Verfahren (d) als Ausgangsstoffe zu verwendenden Hydroxyalkinyl-azolyl-Derivate der allgemeinen Formel Ia sind erfindungsgemässe Verbindungen.

Als Verdünnungsmittel kommen für das erfindungsgemässe Verfahren (a) organische, aprotische Lösungsmittel infrage, wie beispielsweise Diethylether oder Tetrahydrofuran.

Die Umsetzung gemäss Verfahren (a) wird in Gegenwart von aktiviertem Aluminium durchgeführt. Dieses wird durch Zugabe katalytischer Mengen Quecksilber(II)-chlorid und Iod erreicht.

Die Reaktionstemperaturen können bei der Durchführung des erfindungsgemässen Verfahrens (a) in einem grösseren Bereich variiert werden. Im allgemeinen arbeitet man bei Temperaturen zwischen –80 und 100 °C, vorzugsweise zwischen –70 und 60 °C.

Bei der Durchführung des erfindungsgemässen Verfahrens (a) setzt man vorzugsweise auf 1 Mol Azolyl-keton der allgemeinen Formel II 1 bis 2 Mol Propargylhalogenid der allgemeinen Formel III sowie 1 bis 1,5 Mol Aluminium und katalytische Mengen Quecksilber(II)-chlorid und Iod ein. Die Isolierung der Endprodukte erfolgt in allgemein üblicher Weise.

Als Verdünnungsmittel kommen für das erfindungsgemässe Verfahren (b) vorzugsweise polare organische Lösungsmittel infrage. Hierzu gehören vorzugsweise Alkohole, wie Methanol, Ethanol, Propanol, Isopropanol, Butanol oder Isobutanol; Nitrile, wie Acetonitril oder Propionitril; Amide, wie Dimethylformamid, Formamid oder Dimethylacetamid; und Sulfoxide, wie Dimethylsulfoxid oder Sulfolan.

Als Basen kommen für das erfindungsgemässe Verfahren (b) alle üblicherweise verwendbaren anorganischen und organischen Basen in Betracht. Hierzu gehören vorzugsweise Alkalicarbonate, wie Kaliumcarbonat oder Natriumcarbonat; Alkalialkoholate, wie Natriummethylat und -ethylat oder Kaliummethylat und -ethylat; Alkalihydride, wie Natriumhydrid; tertiäre Amine, wie Triethylamin oder Benzyldimethylamin; sowie auch Azole der allgemeinen Formel V oder deren Alkalisalze.

Die Reaktionstemperaturen können bei der Durchführung des erfindungsgemässen Verfahrens (b) in einem grösseren Bereich variiert werden. Im allgemeinen arbeitet man bei Temperaturen zwischen 20 und 150 °C, vorzugsweise zwischen 40 und 120 °C.

Bei der Durchführung des erfindungsgemnässen Verfahrens (b) setzt man vorzugsweise auf 1 Mol Hydroxyalkinyl-halogenid der allgemeinen Formel IV 1 bis 2 Mol Azol der allgemeinen Formel V und 1 bis 4 Mol Base ein. Die Isolierung der Endprodukte erfolgt in allgemein üblicher Weise.

Als Verdünnungsmittel kommen für das erfindungskgemässe Verfahren (c) unter den Reaktionsbedingungen inerte organische Lösungsmittel infrage. Hierzu gehören vorzugsweise Alkohole, wie Methanol, Ethanol, Propanol, Isopropanol, Butanol oder Isobutanol; Nitrile, wie Acetonitril; Ester, wie Essigester; Ether, wie Dioxan; Amide, wie Dimethylformamid; sowie aromatische Kohlenwasserstoffe, wie Benzol und Toluol.

Als Basen kommen für das erfindungsgemässe Verfahren (c) alle üblicherweise verwendbaren anorganischen und organischen Basen in Betracht. Hierzu gehören vorzugsweise die beim Verfahren (b) bereits vorzugsweise genannten Basen.

Die Reaktionstemperaturen können bei der Durchführung des erfindungsgemässen Verfahrens (c) in einem grösseren Bereich variiert werden. Im allgemeinen arbeitet man bei Temperaturen zwischen 20 und 150 °C, vorzugsweise zwischen 40 und 120 °C.

Bei der Durchführung des erfindungsgemässen Verfahrens (c) setzt man vorzugsweise auf 1 Mol Oxiran der allgemeinen Formel VI 1 bis 2 Mol Azol der allgemeinen Formel V und gegebenenfalls katalytische bis molare Mengen Base ein. Die Isolierung der Endprodukte erfolgt in allgemein üblicher Weise.

Als Verdünnungsmittel kommen für das erfindungsgemässe Verfahren (d) Wasser sowie gegenüber Alkalihypohalogenit inerte organische Lösungsmittel infrage. Hierzu gehören beispielsweise Alkohole, wie Methanol oder Ethanol; Ether, wie Diethylether, Dioxan oder Tetrahydrofuran; sowie auch Zweiphasengemische, wie z. B. Ether/Wasser.

Die Reaktionstemperaturen können bei der Durchführung des erfindungsgemässen Verfahrens (d) in einem grösseren Bereich variiert werden. Im allgemeinen arbeitet man bei Temperaturen zwischen 0 und 60 °C, vorzugsweise zwischen 0 und 40 °C.

Bei der Durchführung des erfindungsgemässen Verfahrens (d) setzt man auf 1 Mol der Verbindung der allgemeinen Formel Ia vorzugsweise 1 bis 1,5

Mol Hypohalogenit ein, wobei das Alkalihypohalogenit in situ aus dem entsprechenden Halogen und dem Alkalihydroxid, insbesondere Natrium- und Kaliumhydroxid, erzeugt wird. Die Isolierung der Endprodukte erfolgt in allgemein üblicher Weise.

Zur Herstellung von Säureadditions-Salzen der Verbindungen der allgemeinen Formel I kommen vorzugsweise diejenigen Säure infrage, die bereits im Zusammenhang mit der Beschreibung der erfindungsgemässen Säureadditions-Salze als bevorzugte Säuren genannt wurden.

Die Säureadditions-Salze der Verbindungen der allgemeinen Formel I können in einfacher Weise nach üblichen Salzbildungsmethoden, durch Lösen einer Verbindung der allgemeinen Formel I in einem geeigneten Lösungsmittel und Hinzufügen der Säure, z.B. Chlorwasserstoffsäure, erhalten werden und in bekannter Weise, z.B. durch Abfiltrieren isoliert und gegebenenfalls durch Waschen mit einem inerten organischen Lösungsmittel gereinigt werden.

Zur Herstellung von Metallsalz-Komplexen von Verbindungen der allgemeinen Formel I kommen vorzugsweise Salze von denjenigen Anionen und Kationen in Betracht, die bereits im Zusammenhang mit der Beschreibung der erfindungsgemässen Metallsalz-Komplexe als bevorzugt genannt wurden.

Die Metallsalz-Komplexe von Verbindungen der allgemeinen Formel I können in einfacher Weise nach üblichen Verfahren erhalten werden, so z.B. durch Lösen des Metallsalzes in Alkohol, z.B. Ethanol und Hinzufügen zur Verbindung der allgemeinen Formel I. Man kann Metallsalz-Komplexe in bekannter Weise, z.B. durch Abfiltrieren, isolieren und gegebenenfalls durch Umkristallisation reinigen.

Die erfindungsgemässen Wirkstoffe weisen eine starke mikrobizide Wirkung auf und können zur Bekämpfung von unerwünschten Mikroorganismen praktisch eingesetzt werden. Die Wirkstoffe sind für den Gebrauch als Pflanzenschutzmittel geeignet.

Fungizide Mittel im Pflanzenschutz werden eingesetzt zur Bekämpfung von Plasmodiophoromycetes, Oomycetes, Chytridiomycetes, Zygomycetes, Ascomycetes, Basidiomycetes, Deuteromycetes.

Bakterizide Mittel werden im Pflanzenschutz zur Bekämpfung von Pseudomonoadaceae, Rhizobiaceae, Enterobacteriaceae, Corynebacteriaceae und Streptomycetaceae eingesetzt.

Die gute Pflanzenverträglichkeit der Wirkstoffe in den zur Bekämpfung von Pflanzenkrankheiten notwendigen Konzentrationen erlaubt eine Behandlung von oberirdischen Pflanzenteilen, von Pflanz- und Saatgut und des Bodens.

Als Pflanzenschutzmittel können die erfindungsgemässen Wirkstoffe mit besonders gutem Erfolg zur Bekämpfung von Venturia-Arten, wie gegen den Erreger des Apfelschorfes (Venturia inaequalis), von Erysiphe-Arten, wie gegen den Erreger des echten Gerstenmehltaus (Erysiphe graminis), von weiteren Getreidekrankheiten, wie Cochliobolus sativus und Pyrenophora teres, sowie von Reiskrankheiten, wie Pyricularia oryzae und Pellicularia sasakii, eingesetzt werden. Hervorzuheben ist die zusätzliche bakterizide Wirkung.

Die Wirkstoffe können in die üblichen formulierungen übergeführt werden, wie Lösungen, Emulsionen, Spritzpulver, Suspensionen, Pulver, Stäubemittel, Schäume, Pasten, lösliche Pulver, Granulate, Aerosole, Suspensions-Emulsionskonzentrate, Saatgutpuder, Wirkstoff-imprägnierte Natur- und synthetische Stoffe, Feinstverkapselungen in polymeren Stoffen und in Hüllmassen für Saatgut, ferner in Formulierungen mit Brennsätzen, wie Räuscherpatronen, -dosen, -spiralen u.ä. sowie ULV-Kalt- und Warmnebel-Formulierungen.

Diese Formulierungen werden in bekannter Weise hergestellt, z.B. durch Vermischen der Wirkstoffe mit Streckmitteln, also flüssigen Lösungsmitteln, unter Druck stehenden verflüssigten Gasen und/oder festen Trägerstoffen, gegebenenfalls unter Verwendung von oberflächenaktiven Mitteln, also Emulgiermitteln und/oder Dispergiermitteln und/oder schaumerzeugenden Mitteln. Im Falle der Benutzung von Wasser als Streckmittel können z.B. auch organische Lösungsmittel als Hilfslösungsmittel verwendet werden. Als flüssige Lösungsmittel kommen im wesentlichen in Frage: Aromaten, wie Xylol, Toluol oder Alkylnaphthaline, chlorierte Aromaten oder chlorierte aliphatische Kohlenwasserstoffe, wie Chlorbenzole, Chlorethylene oder Methylenchlorid, aliphatische Kohlenwasserstoffe, wie Cyclohexan oder Paraffine, z.B. Erdölfraktionen, Alkohole, wie Butanol oder Glycol sowie deren Ether, Ketone, wie Aceton, Methylethylketon, Methylisobutylketon oder Cyclohexanon, stark polare Lösungsmittel, wie Dimethylformamid und Dimethylsulfoxid, sowie Wasser. Mit verflüssigten gasförmigen Streckmitteln oder Trägerstoffen sind solche Flüssigkeiten gemeint, welche bei normaler Temperatur und unter Normaldruck gasförmig sind, z.B. Aerosol-Treibgase, wie Halogenkohlenwasserstoffe sowie Butan, Propan, Stickstoff und Kohlendioxid. Als feste Trägerstoffe kommen in Frage: z.B. natürliche Gesteinsmehle, wie Kaoline, Tonerden, Talkum, Kreide, Quarz, Attapulgit, Montmorillonit oder Diatomeenerde und synthetische Gesteinsmehle, wie hochdisperse Kieselsäure, Aluminiumoxid und Silikate. Als feste Trägerstoffe für Granulate kommen in Frage: z.B. gebrochene und fraktionierte natürliche Gesteine wie Calcit, Marmor, Bims, Sepiolith, Dolomit sowie synthetische Granulate aus anorganischen und organischen Mehlen sowie Granulate aus organischem Material, wie Sägemehl, Kokosnussschalen, Maiskolben und Tabakstengel. Als Emulgier- und/oder schaumerzeugende Mittel kommen in Frage: z.B. nichtionogene und anionische Emulgatoren, wie Polyoxyethylen-Fettsäureester, Polyoxyethylen-Fettalkoholether, z.B. Alkylarylpolyglycol-ether, Alkylsulfonate, Alkylsulfate, Arylsulfonate sowie Eiweisshydrolysate. Als Dispergiermittel kommen in Frage: z.B. Lignin-Sulfitablaugen und Methylcellulose.

Es können in den Formulierungen Haftmittel wie Carboxymethylcellulose, natürliche und synthetische pulverige, körnige oder latexförmige Polymere verwendet werden, wie Gummiarabicum, Polyvinylalkohol, Polyvinylacetat.

Es können Farbstoffe wie anorganische Pigmente, z.B. Eisenoxid, Titanoxid, Ferrocyanblau und organische Farbstoffe, wie Alizarin-, Azo- und Metallphthalocyaninfarbstoffe und Spurennährstoffe, wie Salze von Eisen, Mangan, Bor, Kupfer, Kobalt, Molybdän und Zink verwendet werden.

Die Formulierungen enthalten im allgemeinen zwischen 0,1 und 95 Gewichtsprozent Wirkstoff, vorzugsweise zwischen 0,5 und 90%.

Die erfindungsgemässen Wirkstoffe können in den Formulierungen oder in den verschiedenen Anwendungsformen in Mischung mit anderen bekannten Wirkstoffen vorliegen, wie Fungiziden, Bakteriziden, Insektiziden, Akariziden, Nematiziden, Herbiziden, Schutzstoffen gegen Vogelfrass, Wuchsstoffen, Pflanzennährstoffen und Bodenstrukturverbesserungsmitteln.

Die Wirkstoffe können als solche, in Form ihrer Formulierungen oder der daraus durch weiteres Verdünnen bereiteten Anwendungsformen, wie gebrauchsfertige Lösungen, Emulsionen, Suspensionen, Pulver, Pasten und Granulate angewendet werden. Die Anwendung geschieht in üblicher Weise, z.B. durch Giessen, Tauchen, Spritzen, Sprühen, Vernebeln, Verdampfen, Injizieren, Verschlämmen, Verstreichen, Stäuben, Streuen, Trockenbeizen, Feuchtbeizen, Nassbeizen, Schlämmbeizen oder Inkrustieren.

Bei der Behandlung von Pflanzenteilen können die Wirkstoffkonzentrationen in den Anwendungsformen in einem grösseren Bereich variiert werden. Sie liegen im allgemeinen zwischen 1 und 0,0001 Gew.-%, vorzugsweise zwischen 0,5 und 0,001%.

Bei der Saatgutbehandlung werden im allgemeinen Wirkstoffmengen von 0,001 bis 50 g je Kilogramm Saatgut, vorzugsweise 0,01 bis 10 g, benötigt.

Bei Behandlung des Bodens sind Wirkstoffkonzentrationen von 0,00001 bis 0,1 Gew.-%, vorzugsweise von 0,0001 bis 0,02%, am Wirkungsort erforderlich.

Herstellungsbeispiele

Beispiel 1

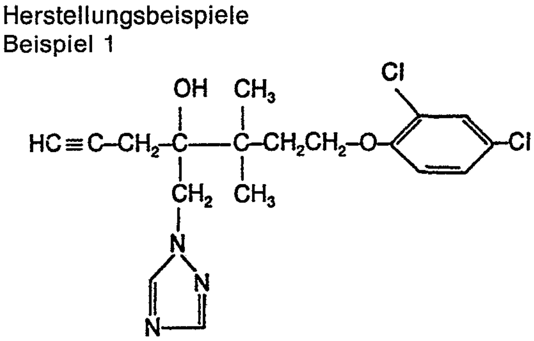

(Verfahren a)

1,58 g (0,058 Mol) Aluminium (Schuppenform) werden mit 7,3 ml Tetrahydrofuran überschichtet und mit einer katalytischen Menge (0,05 g) Quecksilber(II)-chlorid und einem Iodkristall versetzt. Nach 12-stündigem Stehen bei 20 °C werden bei 60 °C 10,3 g (0,087 Mol) Propargylbromid in 11 ml Tetrahydrofuran zugetropft. Man kühlt anschliessend auf −60 °C ab und tropft eine Lösung von 17,1 g (0,05 Mol)

5-(2,4-Dichlorphenoxy)-3,3-dimethyl-1-(1,2,4-triazol-1-yl)-pentan-2-on

in 20 ml Tetrahydrofuran zu. Man lässt auf 0 °C erwärmen, hält noch eine Stunde bei dieser Temperatur und setzt 22 ml einer gesättigten, wässrigen Ammoniumchlorid-Lösung zu. Anschliessend wird filtriert, das Filtrat im Vakuum eingeengt und der Rückstand in 200 ml Essigester aufgenommen. Nach dreimaligem Waschen mit je 100 ml Wasser wird die organische Phase über Natriumsulfat getrocknet und anschliessend im Vakuum eingeengt. Lösungsmittelreste werden bei 50 °C und 0,01 mbar entfernt. Man erhält 14,3 g (74,8% der Theorie)

7-(2,4-Dichlorphenoxy)-5,5-dimethyl-4-(1,2,4-triazol-1-yl-methyl)-1-heptin-4-ol

als bräunliches Öl vom Berechnungsindex $n_D^{20}$ = 1,5642.

Herstellung des Ausgangsproduktes

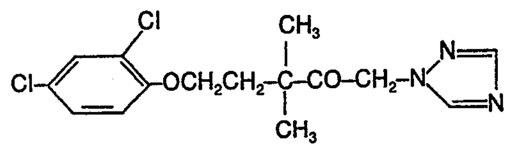

Zu einem Gemisch aus 13,8 g (0,1 Mol) Kaliumcarbonat und 13,8 g (0,2 Mol) 1,2,4-Triazol in 200 ml siedendem Aceton werden 30,9 g (0,1 Mol)

1-Chlor-5-(2,4-dichlorphenoxy)-3,3-dimethyl-pentan-2-on,

gelöst in 80 ml Aceton, unter Rühren zugetropft. Man hält das Gemisch noch 3 Stunden am Sieden, kühlt auf 0 bis 10 °C ab, filtriert vom Salz ab und engt das Filtrat im Vakuum ein. Nach Anreiben des öligen Rückstandes mit etwas Petrolether erhält man 32,4 g (94,6% der Theorie)

5-(2,4-Dichlorphenoxy)-3,3-dimethyl-1-(1,2,4-triazol-1-yl)-pentan-2-on

als farblose Kristalle vom Schmelzpunkt 62 °C.

In analoger Weise und entsprechend den erfindungsgemässen Verfahren (a) bis (d) werden die nachfolgenden Verbindungen der allgemeinen Formel I

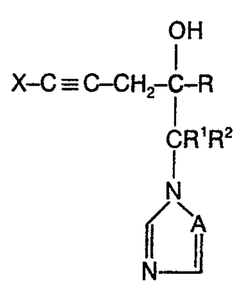 (I)

erhalten:

| Bsp. Nr. | R | $R^1$ | $R^2$ | X | A | Schmelzp. (°C) bzw. Brechungsindex ($n_D^{20}$) |
|---|---|---|---|---|---|---|
| 2 | $-C(CH_3)_2-CH=CH_2$ | H | H | H | N | 1,5220 |
| 3 | $-C(CH_3)_2-CH_2Cl$ | H | H | H | N | 1,5293 |
| 4 | $-C(CH_2Cl)_2CH_3$ | H | H | H | N | 1,5431 |
| 5 | $-C(CH_3)_2-CH_2F$ | H | H | H | N | zähes Öl |
| 6 | $-C(CH_3)_2-O-$ 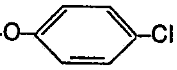 | H | H | H | N | 96–97 |
| 7 | $-C(CH_2F)_2CH_3$ | H | H | H | N | zähes Öl |
| 8 | $-C(CH_3)_2-CH(CH_3)_2$ | H | H | H | N | 1,5171 |
| 9 | $-C(CH_2F)_2CH_3$ | H | H | H | CH | 118–20 |
| 10 | $-C(CH_3)_2-CH_2CH_2$ 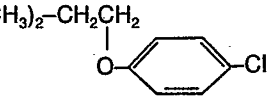 | H | H | H | N | 1,5602 |
| 11 | $-C(CH_3)_2-CH_2CH_2$ 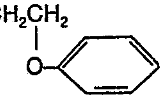 | H | H | H | CH | 69–70 |
| 12 | $-C(CH_3)_2-CH_2CH_2$ 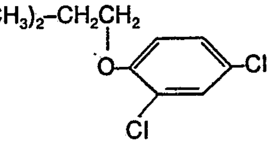 | H | H | H | CH | 133 |
| 13 | $-C(CH_3)_2-CH_2CH_2$ 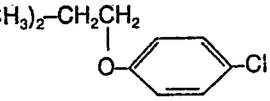 | H | H | H | CH | 85–87 |
| 14 | $-C(CH_3)_2-CH_2$ 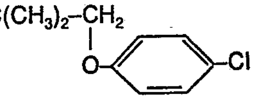 | H | H | H | N | Harz |
| 15 | $-C(CH_3)_2-CH_2F$ | H | H | H | CH | 92–93 |
| 16 | $-C(CH_3)_2-CH_2CH_2$ 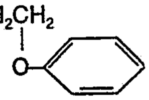 | H | H | H | N | Harz |
| 17 | 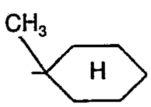 | H | H | H | CH | 139–40 |
| 18 | Adamantyl | H | H | H | CH | 182 |
| 19 | $-C(CH_3)_2-CH_2$<br>$C(CH_3)_3$ | H | H | H | N | $n_D^{25}$ : 1,4928 |
| 20 | Adamantyl | H | H | H | N | 116 |
| 21 | $-C(CH_3)_2-CH_2$<br>$C(CH_3)_3$ | H | H | H | CH | 143 |
| 22 | $-C(CH_3)_2CH_2F$ | 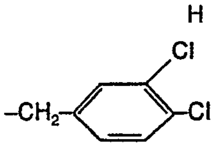 | H | H | N | 128 |
| 23 | $-C(CH_3)_2-CH_2Cl$ | H | H | I | N | 100–02 |
| 24 | $-C(CH_3)_2-O-$ 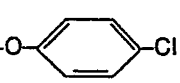 | H | H | I | N | 118–19 |

| Bsp. Nr. | R | $R^1$ | $R^2$ | X | A | Schmelzp. (°C) bzw. Brechungs-index ($n_D^{20}$) |
|---|---|---|---|---|---|---|
| 25 | 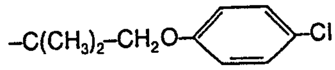 | H | H | I | N | Harz |
| 26 | 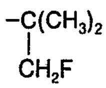 | 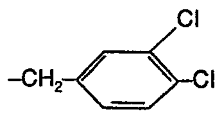 | H | I | N | 151–56 |
| 27 | 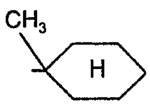 | H | H | H | N | 96–97 |
| 28 | $-C(CH_3)_2-C_3H_7-i$ | H | H | H | CH | 128–31 |
| 29 | 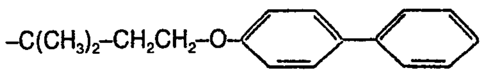 | H | H | H | CH | 159–60 |
| 30 | 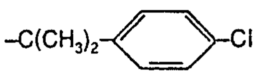 | H | H | H | CH | 136–37 |
| 31 | 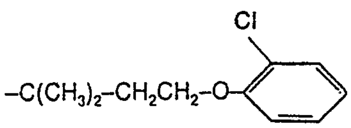 | H | H | H | CH | 104–105 |
| 32 | 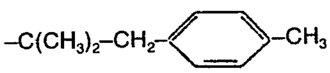 | H | H | H | N | 117–18 |
| 33 | 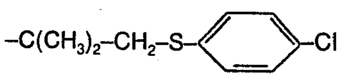 | H | H | H | N | 1,5852 |
| 34 | 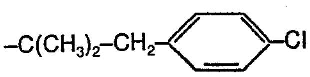 | H | H | H | N | 110–11 |
| 35 | 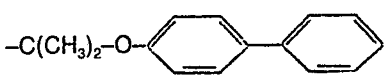 | H | H | H | CH | 118–20 |
| 36 | 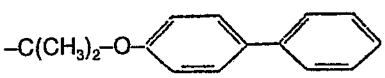 | H | H | H | N | visk. Öl |
| 37 | 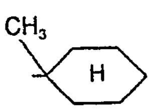 | 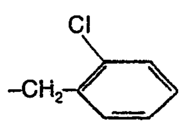 | H | H | N | 120–25 |
| 38 | 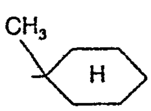 | 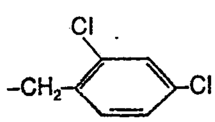 | H | H | N | 94–99 |
| 39 | 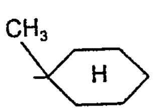 | 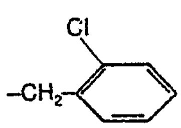 | H | J | N | 128–30 |
| 40 | 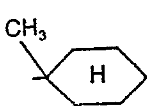 | 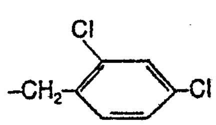 | H | J | N | 119–25 |

| Bsp. Nr. | R | $R^1$ | $R^2$ | X | A | Schmelzp. (°C) bzw. Brechungsindex ($n_D^{20}$) |
|---|---|---|---|---|---|---|
| 41 | 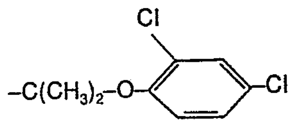 | H | H | H | CH | Harz |
| 42 | 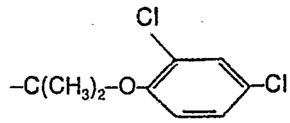 | H | H | H | N | Harz |
| 43 | 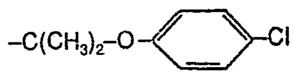 | H | H | H | CH | 96–98 |
| 44 | 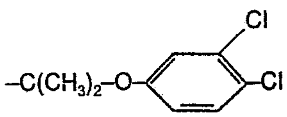 | H | H | H | CH | 106–07 |
| 45 | 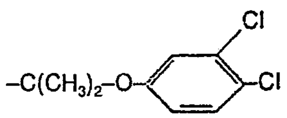 | H | H | H | N | 81–82 |

Verwendungsbeispiele:

In den nachfolgenden Verwendungsbeispielen werden die nachstehend angegebenen Substanzen als Vergleichsverbindungen eingesetzt:

(A) 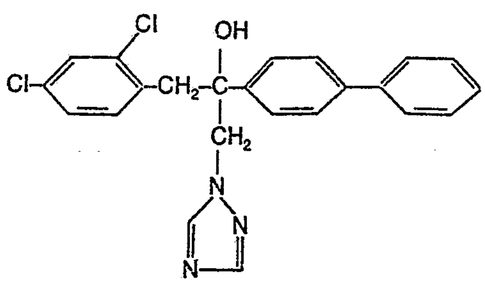

(B) 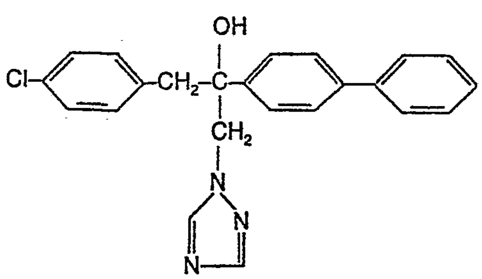

Beispiel A

Venturia-Test (Apfel) / protektiv

Lösungsmittel: 4,7 Gewichtsteile Aceton

Emulgator: 0,3 Gewichtsteile Alkylarylpolyglykolether

Zur Herstellung einer zweckmässigen Wirkstoffzubereitung vermischt man 1 Gewichtsteil Wirkstoff mit den angegebenen Mengen Lösungsmittel und Emulgator und verdünnt das Konzentrat mit Wasser auf die gewünschte Konzentration.

Zur Prüfung auf protektive Wirksamkeit bespritzt man junge Pflanzen mit der Wirkstoffzubereitung bis zur Tropfnässe. Nach Antrocknen des Spritzbelages werden die Pflanzen mit einer wässrigen Konidiensuspension des Apfelschorferregers (Venturia inaequalis) inokuliert und verbleiben dann 1 Tag bei 20 °C und 100% relativer Luftfeuchtigkeit in einer Inkubationskabine.

Die Pflanzen werden dann im Gewächshaus bei 20 °C und einer relativen Luftfeuchtigkeit von ca. 70% aufgestellt.

12 Tage nach der Inokulation erfolgt die Auswertung.

Eine deutliche Überlegenheit in der Wirksamkeit gegenüber dem Stand der Technik zeigen in diesem Test z.B. die Verbindungen gemäss folgendem Herstellungsbeispiel: 10.

Beispiel B

Erysiphe-Test (Gerste) / protektiv

Lösungsmittel: 100 Gewichtsteile Dimethylformamid

Emulgator: 0,25 Gewichtsteile Alkylarylpolyglykolether

Zur Herstellung einer zweckmässigen Wirkstoffzubereitung vermischt man 1 Gewichtsteil Wirkstoff mit den angegebenen Mengen Lösungsmittel und Emulgator und verdünnt das Konzentrat mit Wasser auf die gewünschte Konzentration.

Zur Prüfung auf protektive Wirksamkeit besprüht man junge Pflanzen mit der Wirkstoffzubereitung taufeucht. Nach Antrocknen des Spritzbelages werden die Pflanzen mit Sporen von Erysiphe graminis f.sp.hordei bestäubt.

Die Pflanzen werden in einem Gewächshaus bei einer Temperatur von ca. 20 °C und einer relativen Luftfeuchtigkeit von ca. 80% aufgestellt, um die Entwicklung von Mehltaupusteln zu begünstigen.

7 Tage nach der Inokulation erfolgt die Auswertung.

Eine deutliche Überlegenheit in der Wirksamkeit gegenüber dem Stand der Technik zeigen in diesem Test z.B. die Verbindungen gemäss folgender Herstellungsbeispiele: 6, 4, 8, 10, 14.

Beispiel C
Pellicularia-Test (Reis)
Lösungsmittel: 12,5 Gewichtsteile Aceton
Emulgator: 0,3 Gewichtsteile Alkylarylpolyglykolether

Zur Herstellung einer zweckmässigen Wirkstoffzubereitung vermischt man 1 Gewichtsteil Wirkstoff mit der angegebenen Menge Lösungsmittel und verdünnt das Konzentrat mit Wasser und der angegebenen Menge Emulgator auf die gewünschte Konzentration.

Zur Prüfung auf Wirksamkeit werden junge Reispflanzen im 3- bis 4-Blattstadium tropfnass gespritzt. Die Pflanzen verbleiben bis zum Abtrocknen im Gewächshaus. Anschliessend werden die Pflanzen mit Pellicularia sasakii inokuliert und bei 25 °C und 100% relativer Luftfeuchtigkeit aufgestellt.

5 bis 8 Tage nach der Inokulation erfolgt die Auswertung des Krankheitsbefalles.

Eine deutliche Überlegenheit in der Wirksamkeit gegenüber dem Stand der Technik zeigen bei diesem Test z.B. die Verbindungen gemäss folgender Herstellungsbeispiele: 3, 4, 9, 8, 6, 14, 10, 13 und 26.

**Patentansprüche**

1. Hydroxyalkinyl-azolyl-Derivate der allgemeinen Formel I

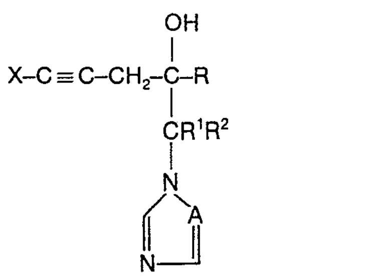

(I)

in welcher

A für ein Stickstoffatom oder eine CH-Gruppe steht,

R für gegebenenfalls einfach bis dreifach, gleich oder verschieden durch Alkyl mit 1 bis 4 Kohlenstoffatomen substituiertes Cycloalkyl mit 3 bis 7 Kohlenstoffatomen, für Adamantyl und

R ferner für die Gruppierungen

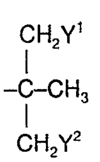 und 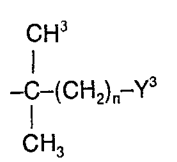 steht, wobei

$Y^1$ für Wasserstoff oder Halogen steht,

$Y^2$ für Halogen steht,

$Y^3$ für Alkyl mit 2 bis 4 Kohlenstoffatomen, Alkoxy und Alkylthio mit jeweils 1 bis 4 Kohlenstoffatomen, Halogenalkoxy und Halogenalkylthio, mit jeweils 1 bis 2 Kohlenstoff- und 1 bis 5 gleichen oder verschiedenen Halogenatomen, Alkenyl mit 2 bis 6 Kohlenstoffatomen, Alkoxycarbonyl mit 1 bis 4 Kohlenstoffatomen im Alkoxyteil, Cyano sowie für gegebenenfalls einfach bis dreifach, gleich oder verschieden substituiertes Phenyl, Phenoxy, Phenylthio, Phenylalkoxy mit 1 bis 4 Kohlenstoffatomen im Alkoxyteil und Phenylalkylthio mit 1 bis 4 Kohlenstoffatomen im Alkylthioteil steht, wobei jeweils als Phenylsubstituenten genannt seien: Halogen, Alkyl mit 1 bis 4 Kohlenstoffatomen; Alkoxy und Alkylthio mit jeweils 1 bis 2 Kohlenstoffatomen; Halogenalkyl, Halogenalkoxy und Halogenalkylthio mit jeweils 1 bis 2 Kohlenstoff- und 1 bis 5 gleichen oder verschiedenen Halogenatomen, Cyclohexyl, Dialkylamino mit 1 bis 4 Kohlenstoffatomen in jedem Alkylteil, Nitro, Cyano, sowie Alkoxycarbonyl mit 1 bis 4 Kohlenstoffatomen im Alkoxyteil; und gegebenenfalls durch Halogen substituiertes Phenyl;

n für die Zahlen 0, 1 oder 2 steht;

$R^1$ für Wasserstoff, geradkettiges oder verzweigtes Alkyl mit 1 bis 4 Kohlenstoffatomen, Alkenyl und Alkinyl mit jeweils 2 bis 4 Kohlenstoffatomen sowie für gegebenenfalls einfach bis dreifach, gleich oder verschieden substituiertes Benzyl, wobei als Phenylsubstituenten die bei $Y^3$ bereits genannten Phenylsubstituenten infrage kommen;

$R^2$ für Wasserstoff oder Methyl steht und

X für Wasserstoff, Brom oder Iod steht,

sowie deren Säureadditions-Salze und Metallsalz-Komplexe.

2. Verbindungen der allgemeinen Formel I in Anspruch 1, wobei

R für jeweils gegebenenfalls einfach oder zweifach, gleich oder verschieden durch Methyl, Ethyl, Isopropyl oder tert.-Butyl substituiertes Cyclopropyl, Cyclopentyl oder Cyclohexyl steht, für Adamantyl steht, ferner die für die Gruppierungen

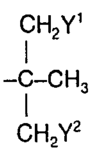 und 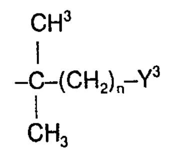 steht, wobei

$Y^1$ für Wasserstoff, Fluor, Chlor oder Brom steht,

$Y^2$ für Fluor, Chlor oder Brom steht,

$Y^3$ für Ethyl, n-Propyl, Isopropyl, tert.-Butyl, Methoxy, Ethoxy, Methylthio, Ethylthio, Trifluormethoxy, Trifluormethylthio, Vinyl, Methoxycarbonyl, Ethoxycarbonyl, Cyano, sowie für gegebenenfalls einfach bis zweifach, gleich oder verschieden substituiertes Phenyl, Phenoxy, Phenylthio, Phenylmethoxy und Phenylmethylthio steht, wobei jeweils als Phenylsubstituenten genannt seien: Fluor, Chlor, Brom Methyl, Ethyl, Isopropyl, tert.-Butyl, Methoxy, Methylthio, Ethoxy, Trifluormethyl, Trifluormethoxy, Trifluormethylthio, Nitro, Dimethylamino, Methoxycarbonyl, sowie gegebenenfalls durch Chlor substituiertes Phenyl,

n für die Zahlen 0, 1 oder 2 steht,

$R^1$ für Wasserstoff, Methyl, Vinyl, Allyl, Propargyl sowie für gegebenenfalls einfach bis zweifach, gleich oder verschieden substituiertes Benzyl steht, wobei als Phenylsubstituenten die bei $Y^3$ bereits genannten Phenylsubstituenten infrage kommen,

A für ein Stickstoffatom oder eine CH-Gruppe steht,

$R^2$ für Wasserstoff oder Methyl steht und

X für Wasserstoff, Brom oder Jod steht.

3. Verfahren zur Herstellung von Hydroxyalkinyl-azolyl-Derivaten der allgemeinen Formel I

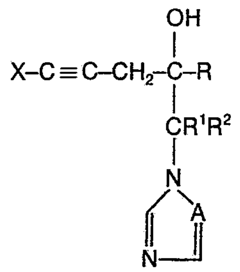 (I)

in welcher

A für ein Stickstoffatom oder eine CH-Gruppe steht,

R für gegebenenfalls einfach bis dreifach, gleich oder verschieden durch Alkyl mit 1 bis 4 Kohlenstoffatomen substituiertes Cycloalkyl mit 3 bis 7 Kohlenstoffatomen, für Adamantyl und

R ferner für die Gruppierungen

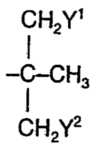 und 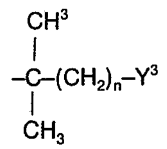

steht wobei

$Y^1$ für Wasserstoff oder Halogen steht,

$Y^2$ für Halogen steht,

$Y^3$ für Alkyl mit 2 bis 4 Kohlenstoffatomen, Alkoxy und Alkylthio mit jeweils 1 bis 4 Kohlenstoffatomen, Halogenalkoxy und Halogenalkylthio, mit jeweils 1 bis 2 Kohlenstoff- und 1 bis 5 gleichen oder verschiedenen Halogenatomen Alkenyl mit 2 bis 6 Kohlenstoffatomen, Alkoxycarbonyl mit 1 bis 4 Kohlenstoffatomen im Alkoxyteil, Cyano sowie für gegebenenfalls einfach bis dreifach, gleich oder verschieden substituiertes Phenyl, Phenoxy, Phenylthio, Phenylalkoxy mit 1 bis 4 Kohlenstoffatomen im Alkoxyteil und Phenylalkylthio mit 1 bis 4 Kohlenstoffatomen im Alkylthioteil steht, wobei jeweils als Phenylsubstituenten genannt seien: Halogen, Alkyl mit 1 bis 4 Kohlenstoffatomen; Alkoxy und Alkylthio mit jeweils 1 bis 2 Kohlenstoffatomen; Halogenalkyl, Halogenalkoxy und Halogenalkylthio mit jeweils 1 bis 2 Kohlenstoff- und 1 bis 5 gleichen oder verschiedenen Halogenatomen, Cyclohexyl, Dialkylamino mit 1 bis 4 Kohlenstoffatomen in jedem Alkylteil, Nitro, Cyano, sowie Alkoxycarbonyl mit 1 bis 4 Kohlenstoffatomen im Alkoxyteil; und gegebenenfalls durch Halogen substituiertes Phenyl;

n für die Zahlen 0, 1 oder 2 steht;

$R^1$ für Wasserstoff, geradkettiges oder verzweigtes Alkyl mit 1 bis 4 Kohlenstoffatomen, Alkenyl und Alkinyl mit jeweils 2 bis 4 Kohlenstoffatomen sowie für gegebenenfalls einfach bis dreifach, gleich oder verschieden substituiertes Benzyl, wobei als Phenylsubstituenten die bei $Y^3$ bereits genannten Phenylsubstituenten infrage kommen;

$R^2$ für Wasserstoff oder Methyl steht und

X für Wasserstoff, Brom oder Iod steht,

sowie von deren Säureadditions-Salzen und Metallsalz-Komplexen

dadurch gekennzeichnet, dass man

a) Azolyl-ketone der allgemeinen Formel II

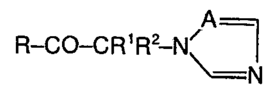 (II)

in welcher

A, R, $R^1$ und $R^2$ die oben angegebene Bedeutung haben,

mit Propargylhalogeniden der allgemeinen Formel III

$$H\equiv C-CH_2-Hal \qquad (III)$$

in welcher

Hal für Chlor oder Brom steht,

in Gegenwart von aktiviertem Aluminium und eines Verdünnungsmittels umsetzt, oder

b) Hydroxyalkinyl-halogenide der allgemeinen Formel IV

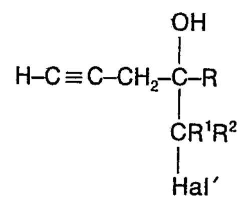 (IV)

in welcher

R, $R^1$ und $R^2$ die oben angegebene Bedeutung haben und

Hal′ für Chlor oder Brom steht,

mit Azolen der allgemeinen Formel V

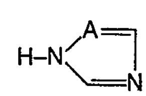 (V)

in welcher

A die oben angegebene Bedeutung hat,

in Gegenwart einer Base und eines Verdünnungsmittel umsetzt, oder

c) Oxirane der allgemeinen Formel VI

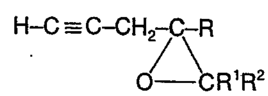 (VI)

in welcher

R, $R^1$ und $R^1$ die oben angegebene Bedeutung haben,

mit Azolen der allgemeinen Formel V

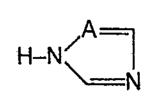 (V)

in welcher

A die oben angegebene Bedeutung hat, in Gegenwart eines Verdünnungsmittels und gegebenenfalls in Gegenwart einer Base umsetzt, oder

d) gegebenenfalls die nach den Verfahren (a), (b) oder (c) erhaltenen Hydroxyalkinyl-azolyl-Derivate der allgemeinen Formel Ia

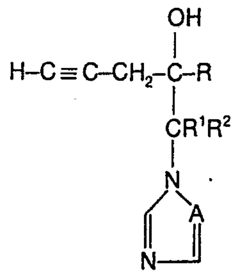

(Ia)

in welcher

A, R, $R^1$ und $R^2$ die oben angegebene Bedeutung haben,

in an sich bekannter Art und Weise mit Alkalihypohalogenit umsetzt, und gegebenenfalls noch an die so erhaltenen Verbindungen der allgemeinen Formel I eine Säure oder ein Metallsalz addiert.

4. Fungizide Mittel, gekennzeichnet durch einen Gehalt an mindestens einem Hydroxyalkinyl-azolyl-Derivat der allgemeinen Formel I in Ansprüchen 1 und 3.

5. Verfahren zur Bekämpfung von Pilzen, dadurch gekennzeichnet, dass man Hydroxyalkinyl-azolyl-Derivate der allgemeinen Formel I auf Pilze oder ihren Lebensraum einwirken lässt.

6. Verwendung von Hydroxyalkinyl-azolyl-Derivaten der allgemeinen Formel I in Ansprüchen 1 und 3 zur Bekämpfung von Pilzen.

7. Verfahren zur Herstellung von fungiziden Mitteln, dadurch gekennzeichnet, dass man Hydroxyalkinyl-azolyl-Derivate der allgemeinen Formel I in Ansprüchen 1 und 3 mit Streckmitteln und/oder oberflächenaktiven Mitteln vermischt.

**Claims**

1. Hydroxyalkinyl-azolyl derivatives of the general formula I

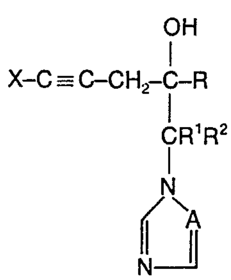

(I)

in which

A represents a nitrogen atom or a CH group,

R represents cycloalkyl which has 3 to 7 carbon atoms and is optionally mono- to trisubstituted by identical or different alkyl radicals having 1 to 4 carbon atoms, or adamantyl and

R furthermore represents the groupings

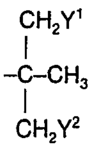

and

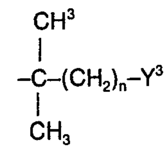

wherein

$Y^1$ represents hydrogen or halogen,

$Y^2$ represents halogen,

$Y^3$ represents alkyl having 2 to 4 carbon atoms, alkoxy and alkylthio, each having 1 to 4 carbon atoms, halogenoalkoxy and halogenalkylthio, each having 1 to 2 carbon and 1 to 5 identical or different halogen atoms, alkenyl having 2 to 6 carbon atoms, alkoxycarbonyl having 1 to 4 carbon atoms in the alkoxy part, cyano and phenoxy, phenylthio, phenylalkoxy having 1 to 4 carbon atoms in the alkoxy part, phenylalkylthio having 1 to 4 carbon atoms in the alkylthio part and phenyl, optionally mono- to trisubstituted by identical or different substituents, the following being mentioned as phenyl substituents in each case: halogen, alkyl having 1 to 4 carbon atoms; alkoxy and alkylthio, each having 1 to 2 carbon atoms; halogenoalkyl, halogenoalkoxy and halogenoalkylthio, each having 1 to 2 carbon and 1 to 5 identical or different halogen atoms, cyclohexyl, dialkylamino having 1 to 4 carbon atoms in each alkyl part, nitro, cyano, and alkoxycarbonyl having 1 to 4 carbon atoms in the alkoxy part; and phenyl which is optionally substituted by halogen; n represents the numbers 0, 1 or 2;

$R^1$ represents hydrogen straight-chain or branched alkyl having 1 to 4 carbon atoms, alkenyl and alkinyl, each having 2 to 4 carbon atoms and benzyl which is optionally mono- to trisubstituted by identical or different substituents, possible phenyl substituents being the phenyl substituents already mentioned under $Y^3$;

$R^2$ represents hydrogen or methyl and

X represents hydrogen, bromine or iodine, and acid addition salts and metal salt complexes thereof.

2. Compounds of the general formula I in Claim 1, wherein

R represents cyclopropyl, cyclopentyl or cyclohexyl, each of which is optionally mono- or disubstituted by identical or different substituents from amongst methyl, ethyl, isopropyl or tert.-butyl, or represents adamantyl or furthermore represents the groupings

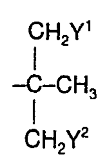

and

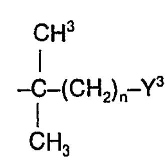

wherein

$Y^1$ represents hydrogen, fluorine, chlorine or bromine,

$Y^2$ represents fluorine, chlorine or bromine,

$Y^3$ represents ethyl, n-propyl, isopropyl, tert.-butyl, methoxy, ethoxy, methylthio, ethylthio, trifluoromethoxy, trifluoromethylthio, vinyl, methoxycarbonyl, ethoxycarbonyl, cyano, and phenoxy, phenylthio, phenylmethoxy, phenylmethylthio and phenyl, optionally mono- to disubstituted by identical or different substituents, the following being mentioned as phenyl substituents in each case: fluorine, chlorine, bromine, methyl, ethyl, isopropyl, tert.-butyl, methoxy, methylthio, ethoxy, trifluoromethyl, trifluoromethoxy, trifluoromethylthio, nitro, dimethylamino, methoxycarbonyl and phenyl which is optionally substituted by chlorine, n represents the numbers 0, 1 or 2

$R^1$ represents hydrogen, methyl, vinyl, allyl, propargyl and benzyl which is optionally mono- to disubstituted by identical or different substituents, possible phenyl substituents being the phenyl substituents already mentioned under $Y^3$,

A represents a nitrogen atom or a CH group,

$R^2$ represents hydrogen or methyl and

X represents hydrogen, bromine or iodine.

3. Process for the preparation of hydroxyalkinylazolyl derivatives of the general formula I

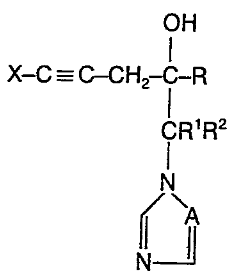

(I)

in which

A represents a nitrogen atom or a CH group,

R represents cycloalkyl which has 3 to 7 carbon atoms and is optionally mono- to trisubstituted by identical or different alkyl radicals having 1 to 4 carbon atoms, or adamantyl and

R furthermore represents the groupings

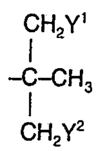 and 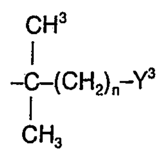 ,

wherein

$Y^1$ represents hydrogen or halogen,

$Y^2$ represents halogen,

$Y^3$ represents alkyl having 2 to 4 carbon atoms, alkoxy and alkylthio, each having 1 to 4 carbon atoms, halogenoalkoxy and halogenalkylthio, each having 1 to 2 carbon and 1 to 5 identical or different halogen atoms, alkenyl having 2 to 6 carbon atoms, alkoxycarbonyl having 1 to 4 carbon atoms in the alkoxy part, cyano and phenoxy, phenylthio, phenylalkoxy having 1 to 4 carbon atoms in the alkoxy part, phenylalkylthio having 1 to 4 carbon atoms in the alkylthio part and phenyl, optionally mono- to trisubstituted by identical or different substituents, the following being mentioned as phenyl substituents in each case: halogen, alkyl having 1 to 4 carbon atoms; alkoxy and alkylthio, each having 1 to 2 carbon atoms; halogenalkyl, halogenoalkoxy and halogenoalkylthio, each having 1 to 2 carbon and 1 to 5 identical or different halogen atoms, cyclohexyl, dialkylamino having 1 to 4 carbon atoms in each alkyl part, nitro, cyano, and alkoxycarbonyl having 1 to 4 carbon atoms in the alkoxy part; and phenyl which is optionally substituted by halogen; n represents the numbers 0, 1 or 2;

$R^1$ represents hydrogen, straigth-chain or branched alkyl having 1 to 4 carbon atoms, alkenyl and alkinyl, each having 2 to 4 carbon atoms and benzyl which is optionally mono- to trisubstituted by identical or different substituents, possible phenyl substituents being the phenyl substituents already mentioned under $Y^3$;

$R^2$ represents hydrogen or methyl and

X represents hydrogen, bromine or iodine,

and acid addition salts and metal salt complexes thereof,

characterised in that

a) azolyl ketones of the general formula II

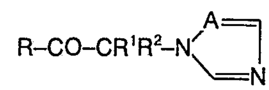

(II)

in which

A, R, $R^1$ and $R^2$ have the abovementioned meaning, are reacted with propargyl halides of the general formula III

$$H{\equiv}C{-}CH_2{-}Hal \qquad (III)$$

in which

Hal represents chlorine or bromine,

in the presence of activated aluminium and a diluent, or

b) hydroxyalkinyl halides of the general formula IV

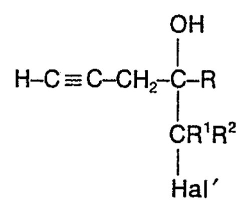

(IV)

in which

R, $R^1$ and $R^2$ have the abovementioned meaning and

Hal′ represents chlorine or bromine,

are reacted with azoles of the general formula V

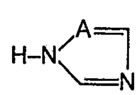

(V)

in which

A has the abovementioned meaning, in the presence of a base and a diluent, or

c) oxiranes of the general formula VI

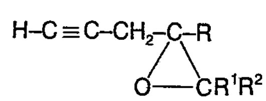

(VI)

in which

R, $R^1$ and $R^2$ have the abovementioned meaning, are reacted with azoles of the general formula V

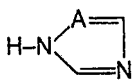

(V)

in which

A has the abovementioned meaning, in the presence of a diluent and, if appropriate, in the presence of a base, or

d) if appropriate, the hydroxyalkinyl-azolyl derivatives obtained by processes (a), (b) or (c), of the general formula Ia

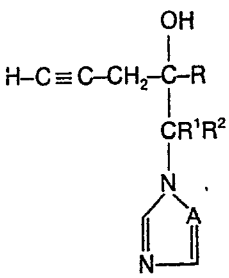

(Ia)

in which

A, R, $R^1$ and $R^2$ have the abovementioned meaning, are reacted with an alkali metal hypohalite in a manner known per se, and, if required, and acid or a metal salt is then added on to the compounds of the general formula I thus obtained.

4. Fungicidal agents, characterised in that they contain at least one hydroxyalkinyl-azolyl derivative of the general formula I in Claims 1 and 3.

5. Process for combating fungi, characterised in that hydroxyalkinyl-azolyl derivatives of the general formula I are allowed to act on fungi or their environment.

6. Use of hydroxyalkinyl-azolyl derivatives of the general formula I in Claims 1 and 3 for combating fungi.

7. Process for the preparation of fungicidal agents, characterised in that hydroxyalkinyl-azolyl derivatives of the general formula I in Claims 1 and 3 are mixed with extenders and/or surface-active agents.

**Revendications**

1. Dérivés hydroxyalcinyl-azolylique de formule générale I:

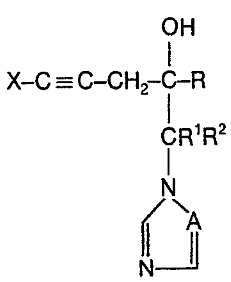

(I)

dans laquelle

A représente un atome d'azote ou un groupe CH,

R un cycloalcoyle ayant 3 à 7 atomes de carbone éventuellement substitué une à trois fois, de manière identique ou différente par un alcoyle ayant 1 à 4 atomes de carbone, un adamantyle et

R en outre les groupements

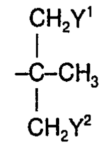

et

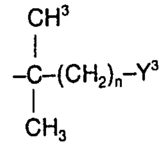

dans lesquels

$Y^1$ représente de l'hydrogène ou de l'halogène

$Y^2$ de l'halogène,

$Y^3$ un alcoyle ayant 2 à 4 atomes de carbone, un alcoxy et alcoylthio ayant chacun 1 à 4 atomes de carbone, un halogénoalcoxy et halogénoalcoylthio ayant chacun 1 à 2 atomes de carbone et 1 à 5 atomes d'halogène identiques ou différents, un alcényle ayant 2 à 6 atomes de carbone, un alcoxycarbonyle ayant 1 à 4 atomes de carbone dans la portion alcoxy, un cyano et même qu'un phényle, phénoxy, phénylthio, phénylalcoxy ayant 1 à 4 atomes de carbone dans la portion alcoxy et phénylalcoylthio ayant 1 à 4 atomes de carbone dans la portion alcoylthio, qui sont éventuellement substitués une à trois fois, de manière identique ou différente, en citant chaque fois comme substituants du phényle:

halogène, alcoyle ayant 1 à 4 atomes de carbone; alcoxy et alcoylthio ayant chacun 1 à 2 atomes de carbone; halogénoalcoyle, halogénoalcoxy et halogénoalcoylthio ayant chacun 1 à 2 atomes de carbone et 1 à 5 atomes d'halogène identiques ou différents, cyclohexyle, dialcoylamino ayant 1 à 4 atomes de carbone dans chaque portion alcoyle, nitro, cyano, de même que alcoxycarbonyle ayant 1 à 4 atomes de carbone dans la portion alcoxy; et phényle éventuellement substitué par de l'halogène,

n représente les nombres 0, 1 ou 2,

$R^1$ de l'hydrogène, un alcoyle à chaîne droite ou ramifiée ayant 1 à 4 atomes de carbone, alcényle et alcinyle ayant chacun 2 à 4 atomes de carbone de même qu'un benzyle éventuellement substitué une à trois fois, de manière identique ou différente, en envisageant comme substituants du phényle les substituants du phényle déjà cités dans les cas de $Y^3$;

$R^2$ de l'hydrogène ou un méthyle et

X de l'hydrogène, du brome ou de l'iode, ainsi que leurs sels d'addition d'acides et complexes avec des sels métalliques.

2. Composés de formule générale I selon la revendication 1, où

R représente un cyclopropyle, cyclopentyle ou cyclohexyle substitué éventuellement chacun une à deux fois, de manière identique ou différente, par un méthyle, éthyle, isopropyle ou t-butyle, un adamantyle, en outre les groupements

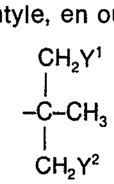

et

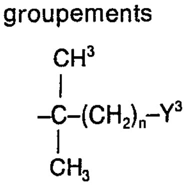

dans lesquels

$Y^1$ représente de l'hydrogène, du fluor, chlore ou brome,

$Y^2$ du fluor, chlore ou brome,

$Y^3$ un éthyle, n-propyle, isopropyle, t-butyle, méthoxy, éthoxy, méthylthio, éthylthio, trifluorométhoxy, trifluorométhylthio, vinyle, méthoxycarbonyle, éthoxycarbonyle, cyano, de même qu'un phényle, phénoxy, phénylthio, phénylméthoxy et phénylméthylthio qui sont éventuellement substitués chacun une à deux fois, de manière identique ou différente, en citant chaque fois comme substituants du phényle: fluor, chlore, brome, méthyle, éthyle, isopropyle, t-butyle, méthoxy, méthylthio, éthoxy, trifluorométhyle, trifluorométhoxy, trifluorométhylthio, nitro, diméthylamino, méthoxycarbonyle, de même qu'un phényle éventuellement substitué par du chlore,

n représente le nombres 0, 1 ou 2,

$R^1$ de l'hydrogène, méthyle, vinyle, allyle, propargyle de même qu'un benzyle éventuellement substitué une à deux fois, de manière identique ou différente, en envisageant comme substituants du phényle les substituants du phényle déjà cités dans le cas de $Y^3$,

A un atome d'azote ou un groupe CH,

$R^2$ de l'hydrogène ou un méthyle et

X de l'hydrogène, du brome ou de l'iode.

3. Procédé de fabrication de dérivés hydroxyalcinyl-azolyliques de formule générale I:

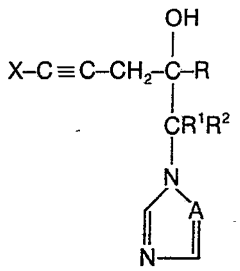
(I)

dans laquelle

A représente un atome d'azote ou un groupe CH,

R un cycloalcoyle ayant 3 à 7 atomes de carbone éventuellement substitué une à trois fois, de manière identique ou différente, par un alcoyle ayant 1 à 4 atomes de carbone, un adamantyle et

R représente en outre les groupements

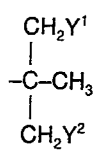 et 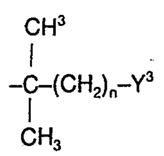

dans lesquels

$Y^1$ représente de l'hydrogène ou de l'halogène,

$Y^2$ de l'halogène,

$Y^3$ un alcoyle ayant 2 à 4 atomes de carbone; un alcoxy et alcoylthio ayant chacun 1 à 4 atomes de carbone, halogénoalcoxy et halogénoalcoylthio ayant chacun 1 à 2 atomes de carbone et 1 à 5 atomes d'halogène identiques ou différents, alcényle ayant 2 à 6 atomes de carbone, alcoxycarbonyle ayant 1 à 4 atomes de carbone dans la portion alcoxy, cyano, de même que phényle, phénoxy, phénylthio, phénylalcoxy ayant 1 à 4 atomes de carbone dans la portion alcoxy et phénylalcoylthio ayant 1 à 4 atomes de carbone dans la portion alcoylthio, qui sont éventuellement substitués chacun une à trois fois, de manière identique ou différente, en citant chaque fois comme substituants du phényle:

halogène, alcoyle ayant 1 à 4 atomes de carbone; alcoxy et alcoylthio ayant chacun 1 à 2 atomes de carbone; halogénoalcoyle, halogénoalcoxy et halogénoalcoylthio ayant chacun 1 à 2 atomes de carbone et 1 à 5 atomes d'halogène identiques ou différents, cyclohexyle, dialcoylamino ayant 1 à 4 atomes de carbone dans chaque portion alcoyle, nitro, cyano, de même que alcoxycarbonyle ayant 1 à 4 atomes de carbone dans la portion alcoxy; et un phényle éventuellement substitué par de l'halogène;

n représente les nombres 0, 1 ou 2,

$R^1$ de l'hydrogène, un alcoyle à chaîne droite ou ramifiée ayant 1 à 4 atomes de carbone, alcényle et alcinyle ayant chacun 2 à 4 atomes de carbone de même qu'un benzyle éventuellement substitué une à trois fois, de manière identique ou différente, en envisageant comme substituants du phényle les substituants du phényle déjà cités dans le cas de $Y^3$,

$R^2$ de l'hydrogène ou un méthyle et

X de l'hydrogène, du brome ou de l'iode,

de même que leurs sels d'addition d'acides et complexes avec des sels métalliques,

caractérisé en ce que

a) on fait réagir des azolyl-cétones de formule générale II:

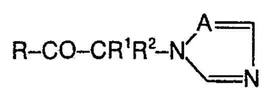
(II)

dans laquelle

A, R, $R^1$ et $R^2$ ont la signification indiquée plus haut,

avec des halogénures de propargyle de formule générale III:

$$H\equiv C{-}CH_2{-}Hal \quad (III)$$

dans laquelle Hal représente du chlore ou du brome,

en présence d'aluminium activé et d'un diluant, ou en ce que

b) on fait réagir des halogénures d'hydroxyalcinyle de formule générale IV:

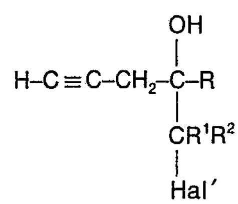
(IV)

dans laquelle

R, $R^1$ et $R^2$ ont la signification indiquée plus haut et

Hal' représente du chlor ou du brome,

avec des azols de formule générale V:

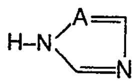

(V)

dans laquelle

A a la signification indiquée plus haut,

en présence d'une base et d'un diluant, ou en ce que

c) on fait réagir des oxiranes de formule générale VI:

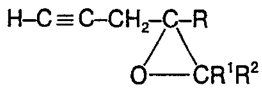

(VI)

dans laquelle

R, $R^1$ et $R^2$ ont la signification indiquée plus haut,

avec des azols de formule générale V:

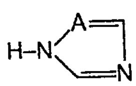

(V)

dans laquelle

A a la signification indiquée plus haut, en présence d'un diluant et éventuellement en présence d'une base, ou en ce que

d) on fait réagir éventuellement des dérivés hydroxyalcinyl-azolyliques obtenus par les procédés (a), (b) ou (c) et de formule générale la:

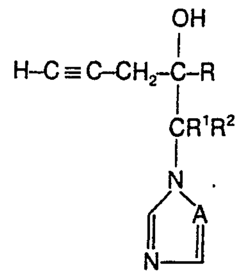

(Ia)

dans laquelle

A, R, $R^1$ et $R^2$ ont la signification indiquée plus haut,

d'une manière connue en elle-même avec un hypohalogénite alcalin, en fixant sur les composés obtenus de formule générale I éventuellement on outre un acide ou un sel métallique.

4. Agents fongicides, caractérisés par une teneur en au moins un dérivé hydroxyalcinyl-azolylique de formule générale I selon les revendications 1 et 3.

5. Procédé pour combattre les champignons, caractérisé en ce qu'on fait agir des dérivés hydroxyalcinyl-azolyliques de formule générale I sur les champignons ou sur leur espace vital.

6. Utilisation de dérivés hydroxyalcinyl-azolyliques de formule générale I selon les revendications 1 et 3 pour combattre les champignons.

7. Procédé de fabrication d'agents fongicides, caractérisé en ce qu'on mélange des dérivés hydroxyalcinyl-azolyliques de formule générale I selon les revendications 1 et 3 avec des diluants et/ou des agents tensioactifs.